(19) Europäisches Patentamt
European Patent Office
Office européen des brevets

(11) EP 1 428 494 A1

(12) **DEMANDE DE BREVET EUROPEEN**

(43) Date de publication:
**16.06.2004 Bulletin 2004/25**

(51) Int Cl.[7]: **A61K 7/032**

(21) Numéro de dépôt: **03293020.8**

(22) Date de dépôt: **03.12.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **13.12.2002 FR 0215871**

(71) Demandeur: **L'OREAL**
**75008 Paris (FR)**

(72) Inventeur: **De La Poterie, Valérie**
**77820 Le Chatelet En Brie (FR)**

(74) Mandataire: **Boulard, Denis**
**L'OREAL - D.I.P.I.**
**25-29 Quai Aulagnier**
**92600 Asnières (FR)**

(54) **Mascara comprenant un polymère filmogène et une phase grasse**

(57) La présente invention a pour objet une composition cosmétique de revêtement des fibres kératiniques comprenant, dans un milieu physiologiquement acceptable :

- une phase dispersante comprenant une phase aqueuse, au moins un polymère filmogène sous forme de particules solides dispersées dans la phase aqueuse et un agent épaississant de ladite phase aqueuse en quantité suffisante telle que la phase dispersante a une viscosité supérieure ou égale à 0,2 Pa.s,
- une phase grasse ayant une viscosité supérieure ou égale à 0,2 Pa.s dispersée dans la phase aqueuse,

la composition ne comprenant pas de tensioactif pour disperser la phase grasse dans la phase dispersante.

L'invention à également pout objet l'utilisation d'un telle composition pour obtenir un film déposé sur les fibres kératiniques lisse et homogène et/ou résistant à l'eau, et/ou aux larmes et/ou à la transpiration et/ou un effet chargeant.

EP 1 428 494 A1

## Description

La présente invention a pour objet une composition cosmétique de revêtement des cils comprenant une phase aqueuse continue comprenant un polymère filmogène et une phase grasse dispersée dans la phase aqueuse. L'invention a également pour objet un procédé de maquillage ou de soin des cils.

La composition peut être une composition de maquillage, encore appelée mascara, une base de maquillage des fibres kératiniques ou base-coat, une composition à appliquer sur un maquillage, dite encore top-coat, ou bien encore une composition de traitement des cils. Plus spécialement, la composition est un mascara.

Il est connu du document WO-A-95/15741 des compositions de mascaras sous forme d'émulsion cire-dans-eau comprenant des tensio-actifs. Toutefois, en raison de la présence de ces tensioactifs, le film de maquillage obtenu avec ces compositions ne présente pas une bonne résistance à l'eau et aux frottements, et le film au contact de l'eau, pendant la baignade ou la douche par exemple, se désagrège en partie en s'effritant ou bien encore en s'étalant autour de l'oeil. L'effritement du film engendre une perte sensible de l'intensité de la couleur du maquillage, obligeant ainsi la consommatrice à renouveler l'application du mascara. L'étalement du film forme, quant à lui, une auréole autour de la zone maquillée très inesthétique. Les larmes et la transpiration provoquent également ces mêmes inconvénients.

Pour favoriser la tenue à l'eau du maquillage, il est connu du document US-A-4423031 d'utiliser des polymères acryliques en dispersion aqueuse. Toutefois, lorsque la composition de mascara comprend des cires émulsionnées dans la phase aqueuse, le dépôt du maquillage ne présente pas une résistance à l'eau suffisante, en raison de la présence de tensioactifs, et se désagrège de la même façon au contact de l'eau ou sous frottements.

Il est également connu de l'art antérieur d'augmenter la teneur en matières sèches (constitué en majeure partie par la phase grasse, notamment les cires) dans une composition de mascara, afin d'obtenir un dépôt de maquillage plus important sur le cil, et donc un maquillage dit chargeant. Cependant, l'utilisation de polymères en dispersion aqueuse ne permet pas d'obtenir un maquillage chargeant des cils car la teneur en particules solides desdites dispersions limite l'augmentation de l'extrait sec en solides dans la composition finale.

Le but de la présente invention est de disposer d'une composition de mascara permettant d'obtenir un maquillage épais (chargeant) des cils et résistant à l'eau froide.

Les inventeurs ont découvert qu'un tel mascara peut être obtenu en utilisant une phase dite dispersante comprenant une phase aqueuse et un polymère filmogène dispersé dans la phase aqueuse, la phase aqueuse étant suffisamment gélifiée pour permettre de disperser une phase grasse, notamment des cires, dans la phase dispersante sans utiliser de tensioactif, ce qui permet d'augmenter sensiblement la teneur en en matières sèches dans le mascara, tout en conservant ses propriétés de résistance à l'eau apportées par le polymère filmogène.

La composition selon l'invention ne comprend donc pas de tensioactif pour disperser toute la phase grasse (c'est à dire tous et indépendemment chacun des constituants de la phase grasse) dans la phase dispersante. Ainsi, par exemple, dans le cas où la phase grasse comprend des cires, la composition est telle qu'elle ne comprend pas de tensioactif pour disperser chacune des cires dans la phase dispersante.

Après l'application de la composition sur les cils, le mascara obtenu est bien résistant à l'eau froide, c'est-à-dire à une eau ayant une température inférieure ou égale à 30 °C, lors de baignade par exemple et/ou aux larmes et/ou à la transpiration ; le mascara est également bien résistant aux frottements, notamment aux frottements des doigts. De plus, le maquillage obtenu épaissit bien les cils et est donc bien chargeant.

De façon plus précise, l'invention a pour objet une composition de revêtement des cils comprenant, dans un milieu physiologiquement acceptable :

- une phase dispersante comprenant une phase aqueuse, au moins un polymère filmogène hydrophobe sous forme de particules solides dispersées dans la phase aqueuse et un agent épaississant de ladite phase aqueuse en quantité suffisante telle que la phase dispersante a une viscosité supérieure ou égale à 0,2 Pa.s,
- une phase grasse ayant une viscosité supérieure ou égale à 0,2 Pa.s, dispersée dans la phase aqueuse,

la composition ne comprenant pas de tensioactif pour disperser la phase grasse dans la phase dispersante.

Par "phase grasse ", on entend, au sens de l'invention, une phase composée d'un ou plusieurs corps non aqueux généralement compatibles entre eux.

La présente invention a également pour objet l'utilisation, dans une composition cosmétique de revêtement des fibres kératiniques comprenant un milieu physiologiquement acceptable, de l'association

- d'une phase dispersante comprenant une phase aqueuse, au moins un polymère filmogène sous forme de particules solides dispersées dans la phase aqueuse et un agent épaississant de ladite phase aqueuse en quantité suffisante telle que la phase dispersante a une viscosité supérieure ou égale à 0,2 Pa.s et
- d'une phase grasse ayant une viscosité supérieure ou égale à 0,2 Pa.s dispersée dans la phase aqueuse,

la composition ne comprenant pas de tensioactif pour disperser la phase grasse dans la phase dispersante, pour obtenir un film déposé sur les fibres kératiniques lisse et homogène et/ou résistant à l'eau, et/ou aux larmes et/ou à la transpiration et/ou un effet chargeant.

L''invention a également pour objet un procédé cosmétique de maquillage ou de soin non thérapeutique des matières kératiniques comprenant l'application sur les matières kératiniques d'une composition telle que définie ci-dessus.

**La phase grasse :**

La phase grasse présente dans la composition a une viscosité, à température ambiante (25°C), supérieure ou égale à 0,2 Pa.s, de préférence supérieure ou égale à 1 Pa. S, et mieux supérieure ou égale à 10 Pa.s. La limite supérieure de la viscosité est atteinte lorsque la phase grasse est sous forme solide et ne présente donc pas de viscosité mesurable.

La mesure de la viscosité est faite au Rhéomat RM 180 équipé d'un mobile MS-r3 ou Ms-r4 tournant à 240 $min^{-1}$ pour une alimentation en courant à 60 Hz ou à 200 $min^{-1}$ pour une alimentation en courant à 50 Hz.

La phase grasse peut comprendre au moins un composé choisi parmi les cires, les gommes, les corps gras pâteux, les polymères semi-cristallins, les huiles, les huiles épaissies par un agent structurant et leurs mélanges.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans la composition selon l'invention peuvent être choisies parmi les cires, solides et rigides, à température ambiante d'origine animale, végétale, minérale ou de synthèse et leurs mélanges. Les cires peuvent avoir un point de fusion allant de 30 °C à 120 °C environ, et mieux allant de 30 °C à 70°C. La cire peut également présenter une dureté allant de 0,5 MPa à 15 MPa, et de préférence allant de 3 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Avantageusement, on utilise une cire polaire. On entend par cire polaire une cire contenant des composés chimiques comportant au moins un groupement polaire. Les groupements polaires sont bien connus de l'homme du métier ; il peut s'agir par exemple de groupement alcool, ester, acide carboxylique. Ne font pas partie des cires polaires les cires de polyéthylène, les cires de paraffine, les cires microcristallines, l'ozokérite, les cires de Fisher-Tropsch.

En particulier, les cires polaires ont un paramètre moyen de solubilité dA de HANSEN à 25 °C tel que dA > 0 $(J/cm^3)^{1/2}$ et mieux dA > 1 $(J/cm^3)^{1/2}$.

$$\delta_a = \sqrt{\delta_p^2 + \delta_h^2}$$

où dP et dH sont respectivement les contributions polaires et de types interactions spécifiques aux paramètres de solubilité de Hansen.

La définition des solvants dans l'espace de solubilité tridimensionnel selon HANSEN est décrite dans l'article de C. M. HANSEN : "The three dimensionnal solubility parameters" J. Paint Technol. 39, 105 (1967) ;

- dH caractérise les forces d'interactions spécifiques (type liaisons hydrogène, acide/base, donneur/accepteur, etc...) ;

- dP caractérise les forces d'interactions de DEBYE entre dipôles permanents ainsi que les forces d'interactions de KEESOM entre dipôles induits et dipôles permanents.

Les paramètres dP et dH sont exprimés en $(J/cm^3)^{½}$.
Comme cire, on peut utiliser la cire d'abeilles, la cire de lanoline, et les cires d'insectes de Chine, la cire de Candellila, la cire d'Ouricurry, la cire de fibres de liège, la cire de canne à sucre, la cire de Berry, la cire du Japon, la cire de sumac; la cire de montan, et les copolymères cireux ainsi que leurs esters.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée, l'huile de lanoline hydrogénée ou encore la cire d'olive obtenue par hydrogénation d'huile d'olive estérifiée avec l'alcool stéarylique telle que la PHYTOWAX Olive 18 L 57 de la société SOPHIM.
On peut encore citer les cires de silicone, les cires fluorées.
On utilise de préférence la cire d'abeille, la cire de Candelilla, la cire de Berry, la cire de jojoba hydrogénée, la cire d'olive obtenue par hydrogénation d'huile d'olive estérifiée avec l'alcool stéarylique et leurs mélanges.
Les gommes sont des corps gras se présentant sous forme de polymère solide à température ambiante ayant un poids moléculaire moyen en poids de 50 000 à 1 000 000. La gomme est souvent vendue en dispersion dans un solvant organique, du type huile de silicone.
La gomme peut être notamment une gomme de silicone répondant à la formule :

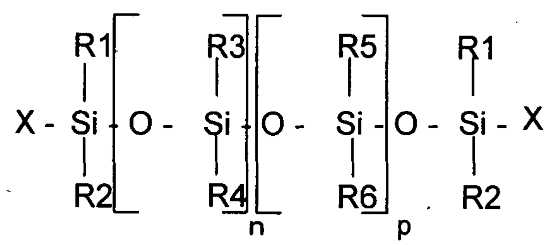

dans laquelle :

$R_1$, $R_2$, $R_5$ et $R_6$ sont, ensemble ou séparément, un radical alkyle ayant 1 à 6 atomes de carbone,
$R_3$ et $R_4$ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.

De manière générale, n et p peuvent prendre des valeurs de 0 à 5000, de préférence de 0 à 3000.
Comme gomme de silicone utilisable selon l'invention, on peut citer celles pour lesquelles :

. les substituants R1 à R6 et X représentent un groupement méthyle, p = 0 et n = 2700, comme celle vendue sous la dénomination SE30 par la société General Electric,
. les substituants R1 à R6 et X représentent un groupement méthyle, p = 0 et n = 2300, comme celle vendue sous la dénomination AK 500000 par la société Wacker,
. les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning.
. les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
. les substituants R1, R2, R5, R6 et X représentent un groupement méthyle, les substituants R3 et R4 représentent un groupement aryle tel que le poids moléculaire du composé soit de 600 000, comme celle vendue sous la dénomination 761 par la société Rhône-Poulenc.

Par "corps gras pâteux", on entend un corps gras ayant une dureté, mesurée à température ambiante, allant de 0,001 à 0,5 MPa, de préférence allant de 0,005 à 0,4 MPa. Un pâteux a en outre un point de fusion compris entre 20 et 60°C, de préférence allant de 25 à 45°C.

Un composé pâteux est un produit visqueux comprenant une fraction solide et une fraction liquide.

A titre d'exemple de corps gras pâteux, on peut citer les polydiméthylsiloxanes (PDMS) ayant des chaînes pendantes du type alkyle ou alcoxy ayant de 8 à 24 atomes de carbone comme le stearyl dimethicone et notamment ceux vendus par Dow Corning sous les références DC2503 ou DC25514 ; les esters d'alcool gras ou d'acide gras ayant de 20 à 55 atomes de carbone (point de fusion allant notamment de 20 °C à 35 °C) comme les esters du cholestérol tels que les triglycérides d'origine végétale hydrogénés comme par exemple l'huile de ricin hydrogénée vendue sous le nom "Thixinr" par la société Rhéox, le polylaurate de vinyle, le propionate d'arachidyle, le triisostearyl or cetyl citrate, le copolymère PVP/Eicosène ; les lanolines et leurs dérivés comme les lanolines acétylées, les lanolines oxypropylènées ou le lanolate d'isopropyle, ayant un point de fusion de 25 à 45°C ; leurs mélanges.

La phase grasse de la composition selon l'invention peut comprendre un polymère semi-cristallin.

Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30°C (notamment allant de 30°C à 70°C), de préférence allant de 30°C à 60°C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.

Les polymères semi-cristallins utilisables dans l'invention sont en particulier :

- les copolymères séquencés de polyoléfines à cristallisation contrôlée, notamment ceux dont les monomères sont décrits dans EP-A-0 951 897.
- les polycondensats et notamment de type polyester, aliphatique ou aromatique ou copolyester aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré (s) tels que décrits dans le document WO-A-01/19333,

et leurs mélanges. Dans ces deux derniers cas, la ou les chaînes latérales ou séquences cristallisables sont hydrophobes.

*A) Polymères semi-cristallins à chaînes latérales cristallisables*

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

. Ces homo- ou co-polymères sont de toute nature du moment qu'ils présentent les conditions indiquées précédemment.

Ils peuvent résulter :

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale, ces polymères sont choisis notamment parmi les homopolymères et copolymères résultant de la polymérisation d'au moins un monomère à chaîne(s) cristallisable(s) qui peut être représenté par la formule X :

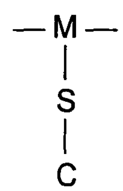

avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe $(CH_2)_n$ ou $(CH_2CH_2O)_n$ ou $(CH_2O)$, linéaire ou ramifié ou cyclique, avec n entier allant de 0 à 22. De préférence « S » est un groupe linéaire. De préférence, « S » et « C » sont différents.

Lorsque les chaînes « -S-C » cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyles en $C_{14}$-$C_{24}$. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en $C_{14}$-$C_{24}$, les (méth)acrylates de perfluoroalkyle avec un groupe alkyle perfluoro en $C_{11}$-$C_{15}$, les N-alkyl (méth)acrylamides avec le groupe alkyle en $C_{14}$ à $C_{24}$ avec ou sans atome de fluor, les esters vinyliques à chaînes alkyle ou perfluoro (alkyle) avec le groupe alkyle en $C_{14}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{14}$ à $C_{24}$ et au moins 6 atomes de fluor pour une chaîne perfluoro alkyle, les alpha-oléfines en $C_{14}$ à $C_{24}$ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :

$\alpha$ ) de Y qui est un monomère polaire ou non polaire ou un mélange des deux :

. Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.

. Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en $C_1$ à $C_{10}$, comme l'$\alpha$-méthylstyrène, un macromonomère du type polyorganosiloxane à insaturation vinylique.

Par "alkyle», on entend au sens au sens de l'invention un groupement saturé notamment en $C_8$ à $C_{24}$, sauf mention exprès, et mieux en $C_{14}$ à $C_{24}$.

β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl (méth)acrylate ou d'alkyl(méth)acrylamide avec un groupe alkyle tel que défini ci-dessus, et notamment en $C_{14}$-$C_{24}$, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

*B) Les polymères portant dans le squelette au moins une séquence cristallisable*

Ces polymères sont notamment des copolymères séquencés constitués d'au moins 2 séquences de nature chimique différente dont l'une est cristallisable.

- On peut utiliser les polymères séquencés définis dans le brevet US-A-5 156 911 ;
- Les copolymères séquencés d'oléfine ou de cyclooléfine à chaîne cristallisable comme ceux issus de la polymérisation séquencée de :

. cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
. avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
. et en particulier les copoly(éthylène/norbornène) blocs et les terpolymères (éthylène/propylène/éthylidène-norbornène) blocs. On peut aussi utiliser ceux résultants de la copolymérisation séquencée d'au moins 2 $\alpha$-oléfines en $C_2$-$C_{16}$ et mieux en $C_2$-$C_{12}$ et encore mieux en $C_4$-$C_{12}$ tels que ceux cités précédemment et en particulier les bipolymères séquencés d'éthylène et d'1-octène.

- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :

. Séquence cristallisable par nature : a) polyester comme les poly(alkylène téréphtalate), b) polyoléfine comme les polyéthylènes ou polypropylènes.
. Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :

α) les copolymères séquencés poly(ε-caprolactone)-b-poly(butadiène), utilisés de préférence hydrogénés, tels que ceux décrits dans l'article "Melting behavior of poly(ε-caprolactone)-block-polybutadiène copolymers" de S. Nojima, Macromolécules, 32, 3727-3734 (1999).

β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).

y) les copolymères séquencés poly(éthylène)-b-copoly(éthylène/propylène) cités dans les articles "Morphology of semi-crystalline block copolymers of ethylene(ethylene-alt-propylene)" de P. Rangarajan et al., Macromolecules, 26, 4640-4645 (1993) et " Polymer agregates with crystalline cores : the system poly(ethylene)-poly(ethylene-propylene)" de P. Richter et al., Macromolécules, 30, 1053-1068 (1997).

δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en $C_{14}$ à $C_{24}$, les (méth)acrylates de perfluoroalkyle en $C_{11}$ à $C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alphaoléfines en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ fluoré ou non fluoré, qui peut être représenté par la formule suivante :

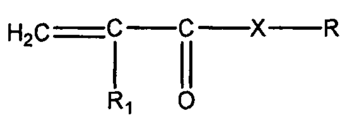

dans laquelle $R_1$ est H ou $CH_3$, R représente un groupe alkyle en $C_1$-$C_{10}$ fluoré ou non fluoré et X représente O, NH ou $NR_2$, où $R_2$ représente un groupe alkyle en $C_1$-$C_{10}$ fluoré ou non fluoré.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment : ceux décrits dans les exemples 3, 4, 5, 7, 9, 13 du brevet US-A-5 156 911 à groupement -COOH, résultant de la copolymérisation d'acide acrylique et d'alkyl(méth)acrylate en $C_5$ à $C_{16}$ et plus particulièrement de la copolymérisation :

- d'acide acrylique, d'hexadécylacrylate et d'isodécylacrylate dans un rapport pondéral 1/16/3,
- d'acide acrylique et de pentadécylacrylate dans un rapport pondéral 1/19,
- d'acide acrylique, d'hexadécylacrylate, éthylacrylate dans un rapport pondéral 2,5/76,5/20,
- d'acide acrylique, d'hexadécylacrylate et de méthylacrylate dans un rapport pondéral 5/85/10,
- d'acide acrylique et de octadécylméthacrylate dans un rapport pondéral 2,5/97,5 ,
- d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le structure « O » de National Starch tel que celui décrit dans le document US-A-5 736 125 de point de fusion 44°C ainsi que les polymères semi-cristallins à chaînes pendantes cristalisables comportant

des groupements fluorés tels que décrits dans les exemples 1, 4, 6, 7 et 8 du document WO-A-01/19333.

On peut encore utiliser les polymères semi-cristallins de bas point de fusion obtenus par copolymérisation d'acrylate de stéaryle et d'acide acrylique ou de NVP tels que décrits dans le document US-A-5 519 063 ou EP-A-550745 et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745 et plus spécialement ceux décrits dans les exemples 3 et 4, ci-après, de préparation de polymère, de température de fusion respectivement de 60°C et 58°C.

De préférence, les polymères semi-cristallins ne comportent pas de groupement carboxylique.

La phase grasse peut également comprendre une huile ou une huile épaissie par un agent structurant choisi les gélifiants lipophiles, les organogélateurs et leurs mélanges, de telle façon que cette phase grasse présente une viscosité supérieure ou égale à 0,2 Pa.s.

Par « huile », on entend un corps gras liquide à température ambiante (25°C) et pression atmosphérique (760mm de Hg soit 105 Pa).

L'huile peut être choisi parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges. Plus précisément, par « huile hydrocarbonée », on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000 mPa.s, de préférence de 50 à 50 000 mPa.s et de préférence encore de 100 à 300 000 mPa.s.

A titre d'exemple d'huile utilisable dans l'invention, on peut citer :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;
- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

L'agent structurant est avantageusement choisi parmi les gélifiants lipophiles, les organogélateurs et leurs mélanges.

Le gélifiant lipophile peut être organique ou minéral, polymérique ou moléculaire.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de distéaryl di-méthyl ammonium.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot,
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974® par la société Degussa, "CAB-O-SIL TS-610® "CAB-O-SIL TS-720® par la société Cabot. La silice pyrogénée hydrophobe présente de préférence une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; l'éthylcellulose comme celles vendues sous le nom d'Ethocel par Dow Chemical ; les polyamides tels que les copolymères d'un diacide en $C_{36}$ condensé sur l'éthylène diamine de masse moléculaire moyenne en poids d'environ 6000 tels que les composés commercialisés par la société Arizona Chemical sous les noms Uniclear 80 et Uniclear 100, les galactommananes comportant de un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et leurs mélanges.

Comme gélifiant lipophile préféré, on utilise des gélifiants organique moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.
Par "phase grasse liquide", on entend, au sens de l'invention, une phase grasse liquide à température ambiante (25° C) et pression atmosphérique (760 mm de Hg, soit 105 Pa), composée d'un ou plusieurs corps gras liquides température ambiante, appelés aussi huiles, généralement compatibles entre eux.

Le réseau supra-moléculaire peut résulter de la formation d'un réseau de fibrilles (dues aux empilements ou agrégations de molécules d'organogélateur), immobilisant les molécules de la phase grasse liquide.
L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.
Les interactions physiques sont diverses mais excluent la co-cristallisation. Ces interactions physiques sont en particulier des interactions du type interactions hydrogènes auto-complémentaires, interactions $\pi$ entre cycles insaturés, interactions dipolaires, liaisons de coordination avec des dérivés organométalliques et leurs associations. En général, chaque molécule d'un organogélateur peut établir plusieurs,types d'interactions physiques avec une molécule voisine. Aussi, avantageusement, les molécules des organogélateurs selon l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6

à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en $C_8$ à $C_{22}$ tels que le dibutylamide de l'acide N-Lauroyl-L-glutamique, fabriqué ou commercialisé par la société Ajinomoto sous la dénomination GP-1 et leurs mélanges.

L'agent structurant peut représenter de 0,01 à 90%, de préférence de 0,5 à 80%, et mieux de 1 à 70% en poids du poids de la phase grasse.

La phase grasse, et notamment la cire et/ou la gomme et/ou le composé pâteux et/ou le polymère semi-cristallin et/ou l'huile et/ou l'huile épaissie par un agent structurant, peut être présente dans la composition selon l'invention en une teneur allant de 5% à 60% en poids, par rapport au poids total de la composition, de préférence allant de 10% à 50% en poids, et préférentiellement allant de 15% à 40% en poids.

Selon un mode préféré de réalisation de l'invention, la phase grasse est formée de particules de cire(s) dispersées dans la phase aqueuse. Aussi, l'invention a pour objet une composition de revêtement des cils comprenant, dans un milieu physiologiquement acceptable, :

- une phase dispersante comprenant une phase aqueuse, un polymère filmogène sous forme de particules solides dispersées dans la phase aqueuse et un agent épaississant de ladite phase aqueuse en quantité suffisante telle que la phase aqueuse a une viscosité supérieure ou égale à 0,2 Pa.s,
- au moins une cire sous forme de particules dispersées dans la phase aqueuse, la composition ne comprenant pas de tensioactif pour disperser la ou les cires dans la phase dispersante.

**Phase dispersante :**

La phase dispersante comprend une phase aqueuse, au moins un polymère filmogène sous forme de particules solides dispersées dans la phase aqueuse et un agent épaississant de ladite phase aqueuse en quantité suffisante telle que la phase dispersante a une viscosité supérieure ou égale à 0,2 Pa.s.

La phase dispersante de la composition a une viscosité supérieure ou égale à 0,2 Pa.s pour permettre de disperser la phase grasse sans utiliser de tensioactif.

Avantageusement, la phase dispersante de la composition a une viscosité allant de 0,2 Pa.s à 50 Pa.s, et de préférence allant de 0,5 Pa.s à 40 Pa.s, et mieux de 1 à 30 Pa.s.

La mesure de la viscosité est faite au Rhéomat RM 180 équipé d'un mobile MS-r3 ou Ms-r4 tournant à 240 $min^{-1}$ pour une alimentation en courant à 60 Hz ou à 200 $min^{-1}$ pour une alimentation en courant à 50 Hz.

Le polymère filmogène est sous forme de particules solides dispersées dans la phase aqueuse. Une telle dispersion de polymère est connue généralement sous le nom de latex ou pseudolatex. Les techniques de préparation de ces dispersions sont bien connues de l'homme du métier.

Par "polymère filmogène", on entend un polymère apte à former à lui seul ou en présence d'agent auxiliaire de filmification, un film continu vu à l'oeil nu et adhérent sur un support comme les matières kératiniques. L'agent auxiliaire de filmification peut être un agent plastifiant bien connu de l'homme du métier.

Le polymère filmogène présent dans la composition selon l'invention peut être choisi parmi les polymères filmogènes hydrophobes ou hydrosoluble.

Par "polymère hydrophobe", on entend un polymère ayant une solubilité dans l'eau à 25 °C inférieure à 1 % en poids.

On utilise de préférence comme polymère filmogène un polymère filmogène hydrophobe : on obtient ainsi un film ayant une bonne résistance à l'eau froide.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523 ® par la société AVECIA-NEORESINS, DOW LATEX 432® par la société DOW CHEMICAL, DAITOSOL 5000 AD® par la société DAITO KASEY KOGYO ou encore le SYNTRAN 5760® par la société INTERPOLYMER.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Comme polyuréthanne filmogène utilisable selon l'invention on peut utiliser ceux commercialisés sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol

propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomere aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

Les particules de polymère filmogène peuvent avoir une taille allant de 5 nm à 600 nm , et de préférence de 20 nm à 300 nm.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes et leurs mélanges. Ces polymères sont généralement appelés polymères hybrides.

Le polymère filmogène en dispersion aqueuse peut être présent dans la composition selon l'invention en une teneur en matières sèches (ou matières actives) allant de 1 % à 60 % en poids par rapport au poids total de la composition, de préférence de 5 % à 40% en poids, et mieux de 10 % à 30 % en poids.

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

De préférence, le polymère filmogène et la phase grasse sont présents en un rapport pondéral polymère filmogène sur phase grasse allant de 0,5 à 1,5 et mieux de 0,5 à 1,3.

La phase aqueuse de la composition peut être constitué essentiellement d'eau. Elle peut comprendre également un mélange d'eau et de solvant miscible à l'eau comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$. La phase aqueuse (eau et éventuellement le solvant organique miscible à l'eau) représente, en pratique, de 5 % à 95% en poids, par rapport au poids total de la composition.

Pour conférer à la phase dispersante la viscosité requise, la phase aqueuse de la composition comprend un agent épaississant permettant d'ajuster la viscosité souhaitée.

Parmi les agents épaississants utilisables selon l'invention, on peut citer :

- les épaississants cellulosiques hydrosolubles tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 4400 H" par la Société Amercol,
- les gommes de guar, notamment celles vendues sous la dénomination VIDOGUM GH 175 par la société UNIPECTINE et sous la dénomination JAGUAR C par la société MEYHALL,
- les gommes de guar quaternisée vendue sous la dénomination de "Jaguar C-13-S" par la Société Meyhall,
- les gommes de guar non-ioniques comprenant des groupements hydroxyalkyle en $C_1$-$C_6$. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120 et JAGUAR HP 105 par la société MEYHALL,

ou sous la dénomination GALACTASOL 40H4FD2 par la société AQUALON.

- les gommes de xanthane, de caroube, de scléroglucane, de gellane, de rhamsan, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polymère acryliques associatifs.

Dans la composition selon l'invention, l'agent épaississant de la phase aqueuse peut être présent en une quantité efficace pour que la phase dispersante présente la viscosité telle que définie précédemment. La teneur en agent épaississant peut par exemple aller de 0,1 % à 15 % en poids, par rapport au poids total de la composition, de préférence de 1 % à 10 %, et mieux de 1 à 5% en poids.

Avantageusement, la composition a une teneur en matières sèches allant de 30 à 60%, de préférence de 35 à 55% et mieux de 40 à 50% en poids par rapport au poids total de la composition.

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec » des compositions selon l'invention, est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition.

Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :

On étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \times (\text{masse sèche} / \text{masse humide}).$$

La composition selon l'invention peut également comprendre une matière colorante comme les matières colorantes pulvérulentes, les colorants liposolubles, les colorants hydrosolubles. Cette matière colorante peut être présente en une teneur allant de 0,1 % à 20 % en poids, par rapport au poids total de la composition, de préférence allant de 1 % à 15 % en poids.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels

que les antioxydants, les conservateurs, les parfums, les neutralisants, les épaississants, les plastifiants, les actifs cosmétiques comme par exemple des émollients, des hydratants, des vitamines, des filtres solaires, et leurs mélanges. Ces additifs peuvent être présents dans la composition en une teneur allant de 0,01 à 10 %, du poids total de la composition.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

L'invention est illustrée plus en détail dans les exemples suivants.

**Exemple 1 :**

On a préparé un mascara ayant la composition suivante :

- Polyuréthane en dispersion aqueuse vendu sous la dénomination AVALURE UR 425 ®, par la société GOODRICH à 49 % en poids de matières actives 18 g MA
- Cire de candellila 15 g
- Hydroxyéthylcellulose 1,9 g
- Pigments 5 g
- Propylène glycol 5 g
- Conservateurs qs
- Eau qsp 100 g

Mode opératoire

L'hydroxyéthylcellulose est dispersée en présence des conservateurs dans l'eau à une température de 80°C, à l'aide d'un agitateur de Raynerie, jusqu'à former un gel. On ajoute ensuite le polyuréthane en dispersion aqueuse en maintenant une température de 70°C, puis les pigments, préalablement dispersés et broyés dans le propylène glycol à l'aide d'une broyeuse tricylindre, sont ajoutés sous agitation. Le mélange est homogénéisé puis la cire préalablement fondue est ajoutée, toujours sous agitation. L'ensemble est refroidi à température ambiante (25°C),

La composition présente un rapport pondéral polymère filmogène sur phase grasse égal à 1,2 et un extrait sec de 44,9%.

La phase dispersante comprenant la dispersion aqueuse de polyuréthane, l'eau et l'épaississant (hydroxyéthylcellulose) présente une viscosité de 3,9 Pa.s, mesurée au Rhéomat RM 180 équipé d'un mobile MS-r3.

Le mascara s'applique facilement sur les cils et forme un maquillage chargeant et résistant à l'eau froide et aux frottements.

**Exemple 2 :**

On a préparé un mascara ayant la composition suivante :

- Polymère acrylique en dispersion aqueuse vendu sous la dénomination SYNTRAN 5760®, à 40% de matières actives par la société INTERPOLYMER 16,5 g MA
- Cire d'abeille 20 g
- Hydroxyéthylcellulose 1 g
- Pigments 5 g
- Propylène glycol 5 g
- Conservateurs qs
- Eau qsp 100 g

On utilise le même mode opératoire que dans l'exemple 1.

La composition présente un rapport pondéral polymère filmogène sur phase grasse égal à 0,825 et un extrait sec de 47,5%.

Ce mascara a été jugé comme épaississant bien les cils et de bonne tenue.

## Revendications

1. Composition de revêtement des cils comprenant, dans un milieu physiologiquement acceptable :

    - une phase dispersante comprenant une phase aqueuse, au moins un polymère filmogène sous forme de particules solides dispersées dans la phase aqueuse et un agent épaississant de ladite phase aqueuse en quantité suffisante telle que la phase dispersante a une viscosité supérieure .ou égale à 0,2 Pa.s,
    - une phase grasse ayant une viscosité supérieure ou égale à 0,2 Pa.s dispersée dans la phase aqueuse,

    la composition ne comprenant pas de tensioactif pour disperser la phase grasse dans la phase dispersante.

2. Composition selon la revendication précédente, **caractérisée en ce que** la phase grasse comprend au moins un composé choisi parmi les cires, les gommes, les corps gras pâteux, les polymères semi-cristallins, les huiles, les huiles épaissies par un agent structurant et leurs mélanges.

3. Composition selon l'une des revendications précédentes, **caractérisée en ce que** la phase grasse comprend une gomme de silicone répondant à la formule

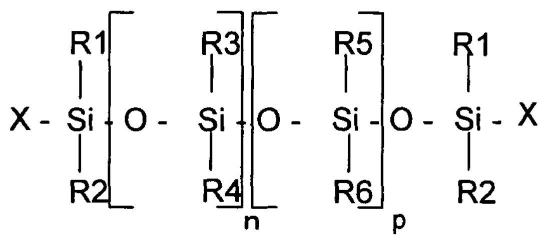

    dans laquelle :

    $R_1$, $R_2$, $R_5$ et $R_6$ sont, ensemble ou séparément, un radical alkyle ayant 1 à 6 atomes de carbone,
    $R_3$ et $R_4$ sont, ensemble ou séparément, un radical alkyle ayant de 1 à 6 atomes de carbone, ou un radical aryle,
    X est un radical alkyle ayant de 1 à 6 atomes de carbone, un radical hydroxyle ou un radical vinyle,
    n et p étant choisis de manière à conférer à la gomme de silicone une viscosité supérieure à 100 000 mPa. s, de préférence supérieure à 500 000 mPa.s,

    avec n et p allant de 0 à 5000, de préférence de 0 à 3000.

4. Composition selon la revendication précédente, **caractérisée en ce que** la phase grasse comprend un corps gras pâteux choisi parmi les polydiméthylsiloxanes ayant des chaînes pendantes du type alkyle ou alcoxy ayant de 8 à 24 atomes de carbone ; les esters d'alcool gras ou d'acide gras ayant de 20 à 55 atomes de carbone, le polylaurate de vinyle, le propionate d'arachidyle, le triisostearyl ou cetyl citrate, le copolymère PVP/Eicosène ; les lanolines et leurs dérivés et leurs mélanges.

5. Composition selon l'une des revendications précédentes, **caractérisée en ce** la phase grasse comprend un polymère semi-cristallin choisi parmi les copolymères résultant de la polymérisation d'au moins un monomère à chaîne cristallisable choisi parmi les (méth)acrylates d'alkyle saturés en $C_{14}$ à $C_{24}$, les (méth)acrylates de perfluoroalkyle en $C_{11}$ à $C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alphaoléfines en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ fluoré ou non fluoré, de formule suivante :

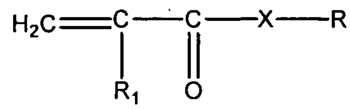

dans laquelle $R_1$ est H ou $CH_3$, R représente un groupe alkyle en $C_1$-$C_{10}$ fluoré ou non fluoré et X représente O, NH ou $NR_2$, où $R_2$ représente un groupe alkyle en $C_1$-$C_{10}$ fluoré ou non fluoré.

**6.** Composition selon l'une des revendications précédentes, **caractérisée en ce que** la phase grasse comprend une huile choisie parmi les huiles hydrocarbonées et/ou siliconées et/ou fluorées volatiles ou non volatiles et leurs mélanges

**7.** Composition selon l'une des revendications précédentes, **caractérisée en ce que** la phase grasse comprend une huile épaissie par un agent structurant.

**8.** Composition selon la revendication précédente, **caractérisée en ce que** l'agent structurant est choisi parmi les gélifiants lipophiles, les organogélateurs et leurs mélanges.

**9.** Composition selon la revendication précédente, **caractérisée en ce que** l'agent structurant représente de 0,01 à 90%, de préférence de 0,5 à 80%, et mieux de 1 à 70% en poids du poids de la phase grasse.

**10.** Composition de revêtement des cils comprenant, dans un milieu physiologiquement acceptable :

- une phase dispersante comprenant une phase aqueuse, au moins un polymère filmogène sous forme de particules solides dispersées dans la phase aqueuse et un agent épaississant de ladite phase aqueuse en quantité suffisante telle que la phase aqueuse a une viscosité supérieure ou égale à 0,2 Pa.s,
- une cire sous forme de particules dispersées dans la phase aqueuse,

la composition ne comprenant pas de tensioactif pour disperser la cire dans la phase aqueuse.

**11.** Composition selon l'une des revendications 2 à 10, **caractérisée en ce que** la cire est choisie parmi la cire d'abeille, la cire de lanoline, les cires d'insectes de Chine, la cire de Candellila, la cire d'Ouricurry, la cire de fibres de liège, la cire de canne à sucre, la cire de Berry, la cire du Japon, la cire de sumac; la cire de montan, les copolymères cireux ainsi que leurs esters, les cires obtenues par hydrogénation catalytique d'huiles animales ou végétales ayant des chaînes grasses, linéaires ou ramifiées, en C8-C32, les cires de silicone, les cires fluorées et leurs mélanges.

**12.** Composition selon l'une des revendications 2 à 11, **caractérisée en ce que** la cire est choisie parmi les cires ayant un point de fusion allant de 30°C à 70°C.

**13.** Composition selon l'une des revendications 2 à 11, **caractérisée en ce que** la cire est une cire polaire.

**14.** Composition selon l'une des revendications précédentes, **caractérisée en ce que** la cire est choisie parmi la cire de candellila, la cire d'abeille, la cire de Berry, la cire de jojoba hydrogénée, la cire d'olive obtenue par hydrogénation d'huile d'olive estérifiée avec l'alcool stéarylique et leurs mélanges.

**15.** Composition selon l'une des revendications précédentes, **caractérisée en ce que** la phase grasse est présente en une teneur allant de 5% à 60% en poids, par rapport au poids total de la composition, de préférence allant de 10% à 50% en poids, et préférentiellement allant de 15% à 40% en poids.

**16.** Composition selon l'une des revendications précédentes, **caractérisée en ce que** les particules de polymère filmogène ont une taille allant de 5 nm à 600 nm, et de préférence allant de 20 nm à 300 nm.

**17.** Composition selon l'une des revendications précédentes, **caractérisée en ce que** le polymère filmogène est choisi parmi les polymères vinyliques, les polyuréthanes, les polyesters, les polymères polyesteramides et/ou les alkydes, et leurs mélanges.

**18.** Composition selon l'une des revendications, **caractérisée en ce que** le polymère filmogène est un polymère filmogène hydrophobe.

**19.** Composition selon l'une des revendications précédentes, **caractérisée en ce que** le polymère filmogène est présent en une teneur en matières sèches allant de 1% à 60 % en poids par rapport au poids total de la composition, de préférence de 5 % à 40% en poids, et mieux de 10 % à 30 % en poids.

**20.** Composition selon l'une des revendications précédentes, **caractérisée en ce que** la phase aqueuse représente de 5 % à 95 % en poids, par rapport au poids total de la composition.

**21.** Composition selon l'une des revendications précédentes, **caractérisée en ce que** la phase dispersante a une viscosité allant de 0,2 Pa.s à 50 Pa.s.

**22.** Composition selon l'une des revendications précédentes, **caractérisée en ce que** l'agent épaississant de la phase aqueuse est choisi parmi les épaississants cellulosiques hydrosoluble, les gommes de guar, les gommes de guar quaternisées, les gommes de guar non-ioniques, les gommes de xanthane, de caroube, de scléroglucane, de gellane, de rhamsan, de karoya, les alginates, la maltodextrine, l'amidon et ses dérivés, l'acide hyaluronique et ses sels, les argiles, les acides polyacryliques réticulés, les polymères poly(métha)crylates de glycéryle, la polyvinylpyrrolidone, l'alcool polyvinylique, les polymères et les copolymères réticulés d'acrylamide, les homopolymères réticulés de chlorure de méthacryloyloxyéthyltriméthylammonium, les polymères associatifs et leurs mélanges.

**23.** Composition selon l'une des revendications précédentes, **caractérisée en ce que** l'agent épaississant de la phase aqueuse est présent en une teneur allant de 0,1 % à 15 % en poids, par rapport au poids total de la composition, de préférence de 1 % à 10 %, et mieux de 1 à 5% en poids.

**24.** Composition selon l'une des revendications précédentes, **caractérisée en ce qu'**elle a une teneur en matières sèches allant de 30 à 60%, de préférence de 35 à 55% et mieux de 40 à 50% en poids par rapport au poids total de la composition.

**25.** Procédé cosmétique de maquillage ou de soin non thérapeutique des matières kératiniques comprenant l'application sur les matières kératiniques d'une composition selon l'une des revendications 1 à 24.

**26.** Utilisation, dans une composition cosmétique de revêtement des fibres kératiniques comprenant un milieu physiologiquement acceptable, de l'association :

- d'une phase dispersante comprenant une phase aqueuse, au moins un polymère filmogène sous forme de particules solides dispersées dans la phase aqueuse et un agent épaississant de ladite phase aqueuse en quantité suffisante telle que la phase dispersante a une viscosité supérieure ou égale à 0,2 Pa.s et
- d'une phase grasse ayant une viscosité supérieure ou égale à 0,2 Pa.s, dispersée dans la phase aqueuse,

la composition ne comprenant pas de tensioactif pour disperser la phase grasse dans la phase dispersante, pour obtenir un film déposé sur les fibres kératiniques lisse et homogène et/ou résistant à l'eau, et/ou aux larmes et/ou à la transpiration et/ou un effet chargeant.

Office européen des brevets

# RAPPORT DE RECHERCHE EUROPEENNE

Numéro de la demande
EP 03 29 3020

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 815 851 A (OREAL)<br>3 mai 2002 (2002-05-03)<br>* exemple 3 *<br>--- | 1-26 | A61K7/032 |
| X | EP 0 875 243 A (OREAL)<br>4 novembre 1998 (1998-11-04)<br>* exemples 2,3 *<br>--- | 1-26 | |
| A | FR 2 818 900 A (OREAL)<br>5 juillet 2002 (2002-07-05)<br>* page 8, ligne 19 - page 9, ligne 32 *<br>* page 16, ligne 12 - ligne 21 *<br>--- | | |
| A | FR 2 823 104 A (OREAL)<br>11 octobre 2002 (2002-10-11)<br>--- | | |
| T | "SYNTRAN TM 5760 MSDS"<br>octobre 2002 (2002-10) ,<br>HTTP://WWW.INTERPOLYMER.COM/ XP002253015<br>----- | | |
| | | | DOMAINES TECHNIQUES RECHERCHES (Int.Cl.7) |
| | | | A61K |

Le présent rapport a été établi pour toutes les revendications

| Lieu de la recherche | Date d'achèvement de la recherche | Examinateur |
|---|---|---|
| MUNICH | 22 mars 2004 | Werner, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 03 29 3020

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-03-2004

| Document brevet cité au rapport de recherche | | Date de publication | Membre(s) de la famille de brevet(s) | | | Date de publication |
|---|---|---|---|---|---|---|
| FR 2815851 | A | 03-05-2002 | FR | 2815851 | A1 | 03-05-2002 |
| | | | CN | 1471386 | T | 28-01-2004 |
| | | | EP | 1331915 | A1 | 06-08-2003 |
| | | | WO | 0234214 | A1 | 02-05-2002 |
| | | | US | 2004022752 | A1 | 05-02-2004 |
| EP 0875243 | A | 04-11-1998 | FR | 2762509 | A1 | 30-10-1998 |
| | | | AT | 202695 | T | 15-07-2001 |
| | | | BR | 9801963 | A | 11-01-2000 |
| | | | CA | 2233349 | A1 | 28-10-1998 |
| | | | DE | 69801024 | D1 | 09-08-2001 |
| | | | DE | 69801024 | T2 | 18-10-2001 |
| | | | EP | 1101488 | A2 | 23-05-2001 |
| | | | EP | 0875243 | A2 | 04-11-1998 |
| | | | ES | 2161020 | T3 | 16-11-2001 |
| | | | JP | 10298034 | A | 10-11-1998 |
| | | | JP | 2000136116 | A | 16-05-2000 |
| | | | PL | 326021 | A1 | 09-11-1998 |
| | | | US | 6235293 | B1 | 22-05-2001 |
| FR 2818900 | A | 05-07-2002 | FR | 2818900 | A1 | 05-07-2002 |
| FR 2823104 | A | 11-10-2002 | FR | 2823104 | A1 | 11-10-2002 |
| | | | EP | 1249225 | A1 | 16-10-2002 |
| | | | JP | 2002322032 | A | 08-11-2002 |
| | | | US | 2002187116 | A1 | 12-12-2002 |

EPO FORM P0460

Pour tout renseignement concernant cette annexe : voir Journal Officiel de l'Office européen des brevets, No.12/82